# EUROPEAN PATENT APPLICATION

(11) **EP 0 903 406 A1**
(43) Date of publication of application: **24.03.1999**
(21) Application number: 98203541.2
(22) Date of filing: 15.04.1991
(51) Int. Cl.: C12N 15/11, C07K 14/16, A61K 31/70

(54) **Modulation of gene expression through interference with RNA secondary structure**

(30) Priority: 04.05.1990 US 518929
(62) Divisional of application: 91920949.4
(71) Applicant: ISIS PHARMACEUTICALS, INC., Carlsbad, CA 92008 (US)
(72) Inventor: Ecker, David J., Leucadia, CA 92024 (US)
(74) Representative: Hallybone, Huw George

(57) **Abstract**

Methods for modulating the expression of genes are disclosed comprising selecting a portion RNA coded by the gene, said RNA portion having subportions forming a secondary structure, and contacting the RNA with oligonucleotide or oligonucleotide analog which can bind with at least one said subportions of the RNA. In accordance with the preferred embodiments, oligonucleotides or oligonucleotide analogs are designed to bind to RNA secondary structures which are of significance to the expression of the gene coding for said RNA. In accordance with a preferred embodiment, methods of treatment of human immunodeficiency virus are similarly disclosed wherein the oligonucleotides or oligonucleotide analogs are targeted at the TAR, CAR or gag-pol elements of HIV.

## Description

### Field of the Invention

This invention relates to the field of therapeutics, particularly the treatment of infections of the human immunodeficiency virus (HIV). It relates to the design, synthesis and application of oligonucleotides and oligonucleotide analogs which inhibit the activity of the HIV and other retroviruses.

### Background of the Invention

This invention relates to materials and methods for modulating the activity of HIV RNA. The invention generally relates to the field of "antisense" compounds, compounds which are capable of specific hybridization with a nucleotide sequence of an RNA. In accordance with preferred embodiments, this invention is directed to methods for achieving therapeutic treatment of disease and regulating gene expression in experimental systems.

It is well known that most of the bodily states in mammals including infectious disease states, are effected by proteins. Such proteins, either acting directly or through their enzymatic functions, contribute in major proportion to many diseases in animals and man. Classical therapeutics has generally focused upon interactions with such proteins in efforts to moderate their disease causing or disease potentiating functions. Recently, however, attempts have been made to moderate the actual production of such proteins by interactions with molecules that direct their synthesis, intracellular RNA. By interfering with the production of proteins, it has been hoped to effect therapeutic results with maximum effect and minimal side effects. It is the general object of such therapeutic approaches to interfere with or otherwise modulate gene expression leading to undesired protein formation.

One method for inhibiting specific gene expression which has been adopted to some degree is the "antisense" approach, where oligonucleotide analogs complementary to a specific, target, messenger RNA, mRNA sequence are used. A number of workers have reported such attempts. Pertinent reviews include C.A. Stein & J.S. Cohen, *Cancer Research,* vol. 48, pp. 2659-2668 (1988) ; J. Walder, *Genes & Development,* vol. 2, pp. 502-504 (1988); C.J. Marcus-Sekura, *Anal. Biochemistry,* vol. 172, 289-295 (1988); G. Zon, *Journal of Protein Chemistry,* vol. 6, pp-131-145 (1987); G. Zon, *Pharmaceutical Research,* vol. 5, pp. 539-549 (1988); A. R. Van der Krol, J.N. Mol, & A.R. Stuitje, *BioTechniques,* vol. 6, pp. 958-973 (1988) and D.S. Loose-Mitchell, *TIPS,* vol. 9, pp. 45-47 (1988). Each of the foregoing provide background concerning general antisense theory and prior techniques.

Prior attempts to inhibit HIV by various antisense approaches have been made by a number of researchers. Zamecnik and coworkers have used phosphodiester oligonucleotides targeted to the reverse transcriptase primer site and to splice donor/acceptor sites P.C. Zamecnik, J. Goodchild, Y. Taguchi, P.S. Sarin, *Proc. Natl. Acad. Sci. USA* 83, 4143 (1986). Goodchild and coworkers have made phosphodiester compounds targeted to the initiation sites for translation, the cap site, the polyadenylation signal, the 5' repeat region and a site between the gag and pol genes. J. Goodchild, S. Agrawal, M.P. Civeira, P.S. Sarin, D. Sun, P.C. Zamecnik, *Proc. Natl. Acad. Sci. U. S. A.* 85, 5507 (1988). In the Goodchild study, the greatest activity was achieved by targeting the polyadenylation signal. Agrawal and coworkers have extended the studies of Goodchild by using chemically modified oligonucleotide analogs which were also targeted to the cap and splice donor/acceptor sites. S. Agrarwal, J. Goodchild, M.P. Civeira, A.H. Thornton, P.S. Sarin, P.C. Zamecnik, *Proc. Nat'l. Acad. Sci. USA* 85, 7079 (1988). A portion of one of these overlapped a portion of the HIV TAR region but was not found to have exemplary effect. Neither was this oligonucleotide analog designed to interfere with the HIV TAR region. Agrawal and coworkers have used oligonucleotide analogs targeted to the splice donor/acceptor site to inhibit HIV infection in early infected and chronically infected cells. S. Agrawal, T. Ikeuchi, D. Sun, P.S. Sarin, A. Konopka, J. Maizel, *Proc. Natl. Acad. Sci. U. S. A.* 86, 7790 (1989).

Sarin and coworkers have also used chemically modified oligonucleotide analogs targeted to the cap and splice donor/acceptor sites. P.S. Sarin, S. Agrawal, M.P. Civeira, J. Goodchild, T. Ikeuchi, P.C. Zamecnik, *Proc. Natl. Acad. Sci. U. S. A.* 85, 7448 (1988). Zaia and coworkers have also used an oligonucleotide analog targeted to a splice acceptor site to inhibit HIV. J.A. Zaia, J.J. Rossi, G.J. Murakawa, P.A. Spallone, D.A. Stephens, B.E. Kaplan, *J. Virol.* 62, 3914 (1988). Matsukura and coworkers have synthesized oligonucleotide analogs targeted to the initiation of translation of the rev gene mRNA. M. Matsukura, K. Shinozuka, G. Zon, et al, *Proc. Natl. Acad. Sci. USA* 84, 7706 (1987); R.L. Letsinger, G.R. Zhang, D.K. Sun, T. Ikeuchi, P.S. Sarin, *Proc. Natl. Acad. Sci. U. S. A.* 86, 6553 (1989). Mori and coworkers have used a different oligonucleotide analog targeted to the same region as Matsukura. K. Mori, C. Boiziau, C. Cazenave, et al., *Nucleic Acids Res.* 17, 8207 (1989). Shibahara and coworkers have used oligonucleotide analogs targeted to a splice acceptor site as well as the reverse transcriptase primer binding site. S. Shibahara, S. Mukai, H. Morisawa, H. Nakashima, S. Kobayashi, N. Yamamoto, *Nucl. Acids Res.* 17, 239 (1989). Letsinger and coworkers have synthesized and tested oligonucleotide analogs with conjugated cholesterol targeted to a splice site. K. Mori, C. Boiziau, C. Cazenave, et al., *Nucleic Acids Res.* 17, 8207 (1989). Stevenson and Iversen have conjugated polylysine to oligonucleotide analogs targeted to the splice donor and the 5'-end of the first exon of the tat gene. M. Stevenson, P.L. Iversen, *J. Gen. Virol.* 70, 2673 (1989).

These prior attempts at targeting HIV have largely focused on the nature of the chemical modification used in the oligonucleotide analog. Although each of the above publications have reported some degree of success in inhibiting some function of the virus, a general therapeutic scheme to target HIV and other retroviruses has not been found. Accordingly, there has been and continues to be a long-felt need for the design of oligonucleotides and oligonucleotide analogs which are capable of effective, therapeutic antisense use.

This long-felt need has not been satisfied by prior work in the field of antisense oligonucleotide therapy for HIV and other retroviruses and viruses. Others have failed to identify target sites in which antisense oligonucleotides or oligonucleotide analogs are therapeutically effective at reasonable rates of application.

### OBJECTS OF THE INVENTION

It is a principal object of the invention to provide therapies for human diseases, particularly the human immunodeficiency virus and other human retroviruses.

It is a further object of the invention to provide molecules, especially oligonucleotides and oligonucleotide analogs which perturb the structure of mRNA.

Yet another object of this invention is to modulate gene expression in cells.

A further object is to interfere with the secondary structure of RNAs through interaction of those structures with oligonucleotides or oligonucleotide analogs.

Another object is to effect such interference through formation of perturbed RNA secondary structures.

Another object is to effect such interference through formation of nucleotide triplexes.

These and other objects of this invention will become apparent from a review of the instant specification.

### SUMMARY OF THE INVENTION

A new paradigm for targeting antisense oligonucleotides to HIV and other retroviruses, viruses and other infectious agents has now been discovered. Prior attempts at antisense targeting to HIV have been focused on inhibition of the synthesis of some particular viral protein thought to be essential to the success of the infection. In the present invention, the same goal (inhibition of viral gene expression) is achieved, but greater, therapeutically significant activity is obtained by targeting particular sites on the HIV or other retrovirus RNA. In the present invention, target RNA structures which have important biological function have been found to be the key target sites. They are interfered with at the level of those structures. It has been determined that targeting these RNA structures is a key to effective antisense therapy with oligonucleotides and oligonucleotide analogs.

In accordance with the present invention, methods of modulating the expression of genes are provided. These comprise selecting or identifying a portion of RNA coded by the gene which has subportions forming a secondary structure. The RNA, or cells containing it, is then contacted with oligonucleotide or oligonucleotide analog which can bind with at least one of the subportions of the RNA. It is preferred that the oligonucleotide or oligonucleotide analog be designed so as to be capable of disrupting the secondary structure of the RNA to effect the inhibition of expression of a gene. The gene is generally one which is believed to give rise to a disease state in an organism and is typically a virus or retrovirus although other infectious organisms can be so attacked.

It is preferred that the oligonucleotide or oligonucleotide analog be capable of binding with at least about six subunits of the RNA subportion. It is more preferred that from eight to fifty units be capable of being bound, with from about 10 to about 20 subunits being even more preferred.

In accordance with preferred embodiments, the oligonucleotide of oligonucleotide analog is capable of forming a duplex structure with the subportion of RNA. Alternatively, and in accordance with certain preferred embodiments, the oligonucleotide or oligonucleotide analog can form a triplex structure with the selected portion of RNA. While the mechanism of the interaction is not known with certainty, it is possible that it may effect modulation of gene expression through a number of ways.

In accordance with preferred embodiments, the RNA portion which is interfered with comprises at least a part of the TAR element of HIV. Other preferred embodiments lead to the interaction of oligonucleotides or oligonucleotide analogs with the CAR element of HIV or with the gag-pol element of HIV.

The oligonucleotides and oligonucleotide analogs in accordance with this invention are themselves believed to be novel. Thus, oligonucleotides which are capable of interacting with subportions of RNA which are capable of forming secondary structures are comprehended. It is also intended that methods of treating animals suspected of having a disease characterized by expression of a gene coding for RNA having a secondary structure may also be provided. Thus, animals suspected of having the disease are contacted with oligonucleotides or oligonucleotide analogs which can bind with the secondary structure of the RNA implicated in the disease process. In particular, the present invention is believed to be effective in the treatment of HIV infections in mammals, especially man. Thus, oligonucleotides or oligonucleotide analogs designed to interact with the TAR, CAR or gag-pol elements of HIV are administered to animals, especially humans suspected of being infected with human immunodeficiency virus.

A host of other viral, retroviral, and other infectious diseases are believed to be amenable to therapeutics in accordance with the present invention.

### BRIEF DESCRIPTION OF THE DRAWINGS

Figure 1A depicts the linear HIV-1 TAR element sequence. Underlined portions connote predicted loops and bulges when the RNA is folded into a hairpin structure. Figure 1B depicts a computer-predicted secondary structure or the HIV-1 TAR element.

Figure 2 depicts the activity of a series of oligonucleotides and oligonucleotide analogs in a cell culture assay for TAR/tat transactivation.

Figure 3 depicts the activity of the oligonucleotide phosphorothioate 94S in a cell culture assay for TAR/tat transactivation.

Figure 4 depicts the activity of the oligonucleotide phosphorothioates 94S and 95S in a cell culture assay for TAR/tat transactivation with a lower dose regimen.

Figure 5 sets forth the partial linear structure of the HIV-1 CAR RNA sequence corresponding to nucleotides 7357-7627.

Figure 6 shows a computer-predicted secondary structure of the HIV-1 CAR element.

Figure 7 is a computer-predicted secondary structure of the gag-pol frame shift region and possible mechanisms of inhibition of frame shifting.

Figure 8 depicts antisense oligonucleotide interference with gag-pol frame shifting.

Figure 9 shows possible interference with gag-pol frame shifting through nucleotide triplex formation.

### DETAILED DESCRIPTION OF PREFERRED EMBODIMENTS

The biological function of RNA is mediated by its structure. mRNA is generally thought of as a linear molecule which contains the information for directing protein synthesis within the sequence of ribonucleotides. Recently, studies have revealed a number of secondary and tertiary structures in mRNA which are important for its function. I. Tinoco, Jr. P.W. Davis, C.C. Hardin, J.D. Puglisi, G.T. Walker, *Cold Spring Harb. Symp. Quant. Biol.* 52, 135 (1987). Secondary structure elements in RNA are formed largely by Watson-Crick type interactions between different regions of the same RNA molecule. Important secondary structural elements include intramolecular double stranded regions, hairpin loops, bulges in duplex RNA and internal loops. Tertiary structural elements are formed when secondary structural elements come in contact with each other or with single stranded regions to produce a more complex three dimensional structure.

Very little is known about the precise three dimensional structure of RNA. However, there have recently been a number of research efforts which have shown that RNA structures, including single stranded, secondary and tertiary structures, have important biological functions beyond simply encoding the information to make proteins in linear sequences. Some of these correlations have been discussed in the following publications: I. Tinoco, Jr., P.W. Davis, C.C. Hardin, J.D. Puglisi, G.T. Walker, *Cold spring Harb. Symp. Quant. Biol.* 52, 135 (1987); O. Resnekov, M. Kessler, Y. Aloni, *J. Biol. Chem.* 264, 9953 (1989); C. Tuerk, P. Gauss, C. Thermes, et al., *Proc. Natl. Acad. Sci. U. S.* A. 85, 1364 (1988); and D.E. Larson, B.H. Sells, *Mol.* Cell. *Biochem.* 74, 5 (1987). Despite the fact that there is little precise structural information on RNA, a number of researchers have measured the binding energies of a large number of RNA duplex structures and have derived a set of rules which can be used to predict the secondary structure of RNA. J.A. Jaeger, D.H. Turner, M. Zuker, *Proc. Natl. Acad. Sci. USA* 86, 7706 (1989); D.H. Turner, N. Sugimoto, Annu. *Rev. Biophys. Biophys. Chem.* 17, 167 (1988). In conjunction with experimental data, these rules are useful in identification of RNA structural elements with important biological function.

It has been discovered to be possible to regulate the activity of RNA in cells by introducing oligonucleotides, or oligonucleotide analogs, which perturb or interfere with the secondary structure of natural RNA's. The oligonucleotides or oligonucleotide analogs interfere with the normal interaction between the RNA and the factors that bind to it. This method Can be used to treat diseases, particularly HIV and other retroviruses. In accordance with the present invention, compositions which bind to biological RNA molecules with significant structural features of biological importance are provided. The present invention employs oligonucleotides and oligonucleotide analogs which bind to these structures. In the context of this invention, the term oligonucleotide refers to a plurality of joined nucleotide units formed from naturally-occurring bases and cyclofuranosyl groups joined by native phosphodiester bonds. This term effectively refers to naturally-occurring species or synthetic species formed from naturally-occurring subunits.

"Oligonucleotide analog," as that term is used in connection with this invention, refers to moieties which function similarly to oligonucleotides but which have non naturally-occurring portions. Thus, oligonucleotide analogs may have altered sugar moieties or inter-sugar linkages. Exemplary among these are the phosphorothioate and other sulfur containing species which are known for use in the art. They may also comprise altered base units or other modifications consistent with the spirit of this invention.

In accordance with certain preferred embodiments, at least some of the phosphodiester bonds of the oligonucleotide have been substituted with a structure which functions to enhance the ability of the compositions to penetrate into the region of cells where the RNA whose activity is to be modulated is located. It is preferred that such linkages be sulfur-containing. It is presently preferred that such substitutions comprise phosphorothioate bonds. Others such as alkyl phosphothioate bonds, N-alkyl phosphoramidates, phosphorodithioates, alkylphosphonates, and short chain alkyl or cycloalkyl structures may also be useful. In accordance with other preferred embodiments, the phosphodiester bonds are substituted with structures which are, at once, substantially non-ionic and non-chiral. Persons of ordinary skill in the art will be able to select other linkages for use in the practice of the invention.

It is generally preferred for use in some embodiments of this invention that the 2' position of the linking sugar moieties in at least some of the subunits of the oligonucleotides or oligonucleotide analogs be substituted. Thus, 2' substituents such as OH, SH, F, OCH₃, OCN, OCHₙCH₃ where n is from 1 to about 20 and other substituents having similar properties may be useful in some embodiments.

Oligonucleotide analogs may also include species which include at least some modified base forms. Thus, purines and pyrimidines other than those normally found in nature may be so employed. Similarly, modifications on the cyclofuranose portions of the nucleotide subunits may also occur as long as the essential tenets of this invention are adhered to.

Such analogs are best described as being functionally interchangeable with natural oligonucleotides (or synthesized oligonucleotides along natural lines), but which have one or more differences from natural structure. All such analogs are comprehended by this invention so long as they function effectively to bind to selected portions of RNA having secondary structure of functional significance.

The oligonucleotides and oligonucleotide analogs in accordance with this invention preferably comprise from about 3 to about 100 subunits. It is preferred that such oligonucleotides and analogs comprise at least about 6 subunits with from about 8 to about 50 subunits being more preferred, and still more preferred to have from about 10 to about 20 subunits. As will be appreciated, a subunit is a base and sugar combination suitably bound to adjacent subunits through phosphodiester or other bonds.

The oligonucleotides and oligonucleotide analogs of this invention can be used in diagnostics, therapeutics and as research reagents and kits. For therapeutic use, the oligonucleotide or oligonucleotide analog is administered to an animal, especially a human, such as are suffering from a virus or retrovirus infection such as AIDS.

It is generally preferred to apply the therapeutic agent in accordance with this invention internally such as orally, intravenously or intramuscularly. Other forms of administration, such as transdermally, topically or intralesionally may also be useful. Inclusion in suppositories may also be useful. Use of the oligonucleotides and oligonucleotide analogs of this invention in prophylaxis is also likely to be useful. Use of pharmacologically acceptable carriers is also preferred for some embodiments. In accordance with the present invention, the oligonucleotides and oligonucleotide analogs which are useful in its performance are best described by the RNA whose secondary structure is to interfered with. Thus, it will be understood by persons of ordinary skill in the art that the oligonucleotides and analogs provided by this invention are those which are capable of binding with RNA having a secondary structure bearing a causal or mediative relationship to a diseased state. All such analogs are comprehended by this invention so long as they bind the target RNA structure at or adjacent to a secondary structure thereof.

A number of RNA secondary structures have recently been identified for which application of this invention will likely provide therapeutic utility. Others will also be useful as well. Some of these include the HIV TAR structures; S. Feng, E.C. Holland, *Nature* 334, 165 (1988), including the stem loops at nucleotide 1-59, and 60-104 according to the nucleotide sequence as described by Ratner L. Ratner L.; W. Haseltine, R. Patarca, K.J. Livak, B. Starcich, S.F. Josephs, *Nature* 313, 277 (1985); the boundary between the EGP/OMP regions of HIV, S. Le, J. Chen, M.J. Braun, M.A. Gonda, J.V. Maizel, *Nucl. Acids Res.* 16, 5153 (1988); the boundary between the TMP/env genes of HIV, S. Le, J. Chen, M.J. Braun, M.A. Gonda, J.V. Maizel, *Nucl. Acids Res.* 16, 5153 (1988); the HIV CAR structure, E.T. Dayton, D.M. Powell, A.I. Dayton, *Science* 246, 1625 (1989); the stem loop structure at the junction between the HIV gag and pol genes (nucleotides 1629-1674); the HIV CRS element; and the human iron responsive element (IRE) J.L. Casey, M.W. Hentze, D.M. Koeller, et al, *Science* 240, 924 (1988).

In addition, there are regions of RNA which are primarily thought of as single stranded areas which have been identified as sites for protein binding. For example, the sequence 5'-AUUUA-3' has been identified as a signal for a protein to bind which leads to degradation of RNA. J.S. Malter, Science 246, 664 (1989). The structure of this region in not known. However, that does not preclude the practice of this invention with this sequence. Additional RNA elements, with as yet unknown structures, can also be the subject of this invention.

It is not absolutely necessary to know the actual RNA structure in order to practice this invention, it is only necessary to know that a specific RNA sequence is recognized by an RNA binding element and that this interaction has important biological consequences. In this regard, the viral RNA sequences and structures which are recognized by the structural proteins of retroviruses for virion formation may be the subject of this invention as may many others. It is not intended that application of this invention be limited to presently known structures. Binding to any RNA structure which has an important biological function falls within the spirit and scope of this invention.

This disclosure provides several methods to interfere with the natural function of an RNA structural element and others will be apparent to persons skilled in the art. By using the rules of Watson-Crick hybridization and free energy predictions for hybridization of oligonucleotides or oligonucleotide analogs designed to be complementary to RNA which comprises secondary structures it has now been found that the oligonucleotides and oligonucleotide analogs will compete with internal RNA structures by forming stable heteroduplexes. The energy barriers to heteroduplex formation are overcome by designing oligonucleotides or oligonucleotide analogs to form a more stable heteroduplex than the internal RNA structure on the target RNA.

Kinetic considerations for strand invasion of an existing RNA duplex also enter into the oligonucleotide design. It is possible to disrupt an existing RNA secondary structure with an invading strand by designing the invading strand to have at least three, and preferably more bases complementary to regions to the target RNA which are not involved in base pairing. This provides a kinetic "foothold" for the invading strand to initiate the process of heteroduplex formation.

It is also disclosed that duplex RNA structure can be perturbed by binding to it by triple strand formation. In contrast to heteroduplex formation, where the RNA secondary structure is broken by an invading strand, triple strand formation generally preserves the existing RNA duplex hydrogen bonding pattern, but binds in a helical groove with additional hydrogen bonds. Triple strand formation is a phenomenon which has been known in a limited sense for some time. General reviews which describe triple strand formation with duplex DNA include; J.C Hanvey, N. Shimizu, R.D. Wells, *Proc. Natl. Acad. Sci. USA* 85, 6292 (1988); and S. Arnott, E. Selsing, J. *Mol. Biol.* 88, 509 (1974). Triple strand formation with RNA homopolymers has been previously described in S.L. Broitman, D.D. Im, J.R. Fresco, *Proc. Natl. Acad. Sci. USA* 84, 5120 (1987). It has not, however, previously been disclosed to inhibit the function of RNA by binding to duplex regions with oligonucleotides or oligonucleotide analogs by triple strand formation. It is now believed, however, that binding to regions of RNA secondary structure, such as in the stem regions of stem-loops, will perturb the interactions between the natural RNA and the factors which bind to it, thus modulating gene expression.

In accordance with the present invention, it will be understood that the term "to bind" as it refers to the interaction between an oligonucleotide or oligonucleotide analog and an RNA portion or subportion may have any of several, related meanings. Thus, the present invention comprehends binding of an oligonucleotide or analog with at least one of subportions forming a secondary structure of an RNA portion comprising them. It will be understood that the oligonucleotide or analog will bind with at least one of the subportions of the RNA portion in a Watson-Crick fashion so as to form, locally, a heteroduplex between the RNA subportion and the oligonucleotide or analog. This heteroduplex formation is believed to result in alteration of the secondary structure of the RNA portion. The exact mechanism and the result of this effect is not known with certainty, yet it is believed that the normal secondary structure of the RNA portion is gradually replaced by the binding of the oligonucleotide with one or more of the subportions of the RNA portion. Since the electronic and steric factors which attend the new heteroduplex are different from those of the natural-occurring RNA portion, the effectiveness and nature of the function to generate protein from the RNA is interfered with. The resulting formation of defective or missing protein manifests itself overall as a modulation in the expression of the gene coding for the RNA.

The present invention also comprehends the formation of triplexes with RNA portions having secondary structures. Once again, the precise nature of such triplexes is not fully understood however it is believed that suitably-constructed oligonucleotides or oligonucleotide analogs can so interact with portions of RNA having a secondary structure in some circumstances. The resulting triplex formation is believed to grossly interfere with translation of protein from the RNA thus leading to modulation of expression of the gene from which the RNA derives.

In accordance with the invention it is not necessary that the interaction of oligonucleotide or oligonucleotide analog with the RNA portion or subportion -- the binding of the two -- result in either non-formation or malformation of protein. It may be in some circumstances that interruption of some control or other function having a significant role in the gene expression protocol may be an effective means of modulating that expression. One example of this relates to the gag-pol locus in HIV. Thus, it is not necessary that protein translation be stopped or that defective proteins be produced. Rather, through interference of the gag-pol region it is believed possible to interfere with frame-shifting which is believed to lead to the preparation of fusion proteins of significant importance to the HIV organism.

In short, any interaction or binding of oligonucleotide or oligonucleotide analog with an RNA having a secondary structure is believed to have the potential for interference with RNA function and, hence, for modulation of the expression of the gene from which the RNA derives. It is likely that persons of ordinary skill in the art will find other means of interfering with RNA secondary structures other than those set forth with specificity herein. All such means are, however, contemplated by the present invention.

While a wide variety of oligonucleotides and oligonucleotide analogs are believed to be useful in practice of the present invention, it has been found to be preferred to design such oligonucleotides and analogs so as to bind with at least about six subunits of a subportion of an RNA portion having a secondary structure. In accordance with other preferred embodiments, oligonucleotides which combine with from about six to about 30 and even more preferably with about 10 to about 20 subunits are preferred. As discussed above, it is presently believed that the TAR element of HIV is an excellent target for employment of the present invention. Accordingly, preparation of oligonucleotide or oligonucleotide analog for binding with one or more subportions of the TAR region of HIV are preferred. A similar consideration attends the interference with the functioning of the CAR element of HIV. Accordingly, preparation of oligonucleotides or oligonucleotide analogs which interfere with that portion are also preferred.

In a similar fashion, interference with gag-pol element of HIV may also be preferred in accordance with the practice of certain embodiments of this invention. In such case, it is expected that frame-shifting will be interfered with leading to the malformation or non-formation of essential proteins of the gag-pol family.

Therapeutics are particular objects of the present invention. Thus, presenting oligonucleotides and oligonucleotide analogs in accordance with the present invention in pharmaceutically acceptable carriers may be highly useful. It is desired to treat animals suspected of having diseases characterized by expression of genes coding for RNA having secondary structures. Thus, animals suspected of having such diseases are contacted with oligonucleotides or oligonucleotide analogs which are designed to bind with a secondary structure of those RNAs. This is especially true for treatment of the disease AIDS. In such case, it is presently preferred to employ oligonucleotides or analogs which are targeted at the TAR, CAR or gag-pol elements of HIV.

Overall, it is preferred to administer to patients suspected of suffering from the foregoing disease states with amounts of oligonucleotide or analog, in either native form or suspended in a carrier medium in amounts and upon treatment schedules which are effective to reduce the symptomology of that disease. It is within the scale of a person's skill in the art to determine optimum dosages and treatment schedules for such treatment regimens.

### EXAMPLE 1. The HIV TAR Element

An elaborate set of control elements in the HIV genome determine whether the virus replicates or remains dormant. Of the nine genes identified in the HIV genome, only three are from the core and envelope. W.A. Haseltine, F. Wong-Staal, *Scientific American* October, 52 (1988). The other six genes are involved in regulation of the production of viral proteins. Regulatory genes work by encoding a protein that interacts with a responsive element somewhere else on the viral genome. The major regulatory gene responsible for initiating the burst of replication is the tat (transactivator) gene. The product of the tat gene, tat protein, works by interaction with a short sequence element known as TAR (trans-acting responsive element). The TAR sequence is encoded in the viral long terminal repeats (LTR's), and therefore is included in the mRNA from every HIV gene.

Expression of the tat protein results in increased expression of other HIV genes up to 1,000 fold, including the tat gene itself. Because of this autoregulatory positive feedback, and the fact that the TAR sequence is included in the mRNA from every HIV transcript, an immense amount of viral gene expression is triggered when the tat gene is activated. The interaction between the tat gene and the TAR element is therefore crucial to the life cycle of the HIV, and specific disruption of this interaction is likely to interrupt the propagation of the virus.

The mechanism of trans-activation of TAR-containing genes by the tat protein has recently been studied intensely. Phillip, A. Sharp, Robert, A. Marciniak, *Cell* 59, 229 (1989). Although much remains to be learned, two important points have become clear; that tat increases the expression of TAR-containing genes by increasing both the amount of viral mRNA and the efficiency of its translation, and that TAR functions as an RNA structure, rather than a DNA structure.

The unusual conclusion that tat increases the transcription of TAR-containing genes, but does so by interacting with the TAR element in RNA was derived from a number of observations. Phillip, A. Sharp, Robert, A. Marciniak, *Cell* 59, 229 (1989). In order to achieve trans-activation, the TAR element must be located immediately downstream from the site of initiation of transcription. Moreover, TAR is orientation dependent; if inserted in the inverse orientation, it fails to function.

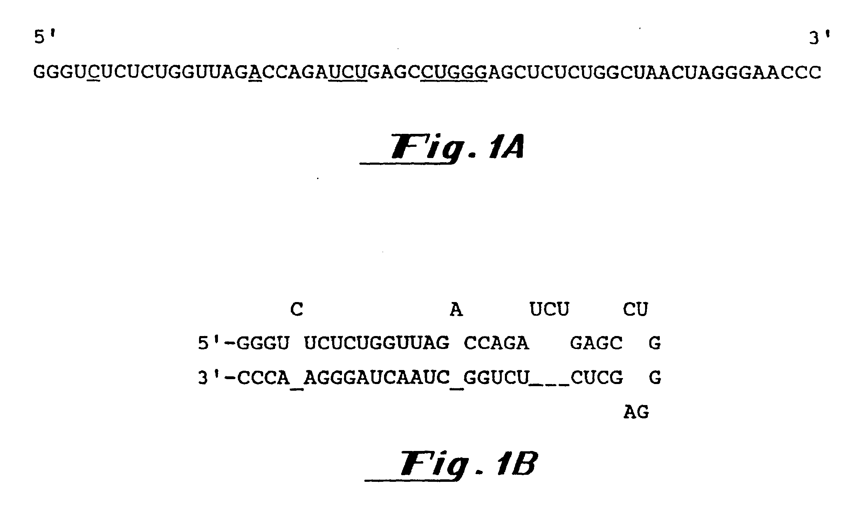

Some of the strongest evidence that tat interacts with TAR as an RNA structure has come from mutagenesis experiments. Efforts to study the TAR element were stimulated by the observation that the tat protein from HIV-1 was capable of trans-activating vectors containing the TAR region of HIV-2, a different strain of virus, even though there is very little primary sequence homology in the TAR region between the two strains. S. Feng, E.C. Holland, *Nature 334, 165* (1988). However, examination of the TAR sequence from HIV-1 and HIV-2 with computer programs that predict RNA secondary revealed the potential of RNA stem-loop structures, with a single stem-loop in the TAR region of HIV-1 and three stem-loop structures in HIV-2. Although the compositions and lengths of the stems were divergent, all four loops contained the pentanucleotide CUGGG as shown in Figures 1A and lB. Mutagenesis experiments revealed that each of the nucleotides present in the loop are absolutely essential for trans-activation by tat, but that base substitutions in the stem were tolerated to some extent so long as the stem structure was maintained. S. Feng, E.C. Holland, *Nature* 334, 165 (1988).

Further evidence for the TAR structure functioning as RNA was obtained from experiments in which the sequences flanking the stem-loop structure were altered creating competing secondary structures in the RNA that were more stable than the natural TAR stem-loop. B. Berkhout, *Cell* 59, 273 (1989). This was accomplished by introducing additional sequences into the TAR-containing RNA that were antisense to the 5' side of the stem-loop structure. Trans-activation of the modified TAR structure was lost, suggesting that the TAR sequences alone are not sufficient for trans-activation, but that these sequences must fold up in the proper secondary structure to be active. It also suggests that antisense sequences to the TAR stem-loop are capable of disrupting the natural RNA structure.

Direct biochemical evidence for TAR stem-loop structure has also been obtained. The TAR RNA has been enzymatically synthesized *in vitro* and probed with enzymes which selectively cleave single stranded regions of RNA, but not duplex structures. The results of the enzyme cleavage patterns were consistent with the computer predicted RNA secondary structure. B. Berkhout, *Cell* 59, 273 (1989).

In summary, there is strong and direct evidence from a number of studies that the HIV tat protein is responsible for triggering an enormous amount of viral gene expression, that this occurs by interaction with the TAR sequence which is incorporated into every HIV mRNA transcript, that the HIV TAR sequence functions as an RNA structure and that the correct TAR RNA structure is essential for tat trans-activation.

It has now been discovered that compounds which specifically bind the TAR RNA structure and interfere with tat trans-activation have activity as therapeutic agents for HIV infection. It is intended that all strains of HIV fall within the spirit and scope of this invention. Different strains of HIV may have different TAR sequences which will therefore fold into different structures. This invention can be practiced on alternative strains of HIV by changing the sequence of the oligonucleotide or oligonucleotide analog to complement the structure of the alternative strain.

TAR and tat function has been studied by removing the genes from the HIV genome and studying them in cell lines in isolation. Vectors have been constructed to study the interactions between the tat protein and TAR element. The tat gene is expressed under the SV40 promoter. The TAR region is expressed from a separate plasmid fused to an easily assayed reporter gene, the placental alkaline phosphatase gene (PAP). P. Henthorn, P. Zervos, N. Raducha, H. Harris, T. Kadesch, *Proc. Natl. Acad. Sci. USA* 85, 6342 (1988). Enzymatic activity in cell culture models has been shown to be dependent upon both the presence of the essential elements of the TAR region and the presence of the tat protein. P. Sharp, R. Marciniak, *Cell* 59, 229 (1989); S. Feng, E.C. Holland, *Nature* 334, 165 (1988); Michael, F. Laspia, Andrew, P. Rice, Michael, B. Mathews, *Cell* 59, 283 (1989); J.A. Garcia, D. Harrich, E. Soultanakis, F. Wu, R. Mitsuyasu, R.B. Gaynor, *EMBO J.* 8, 765 (1989); and B. Berkhout, *Cell* 59, 273 (1989). In essence, the vector system reconstitutes the events of tat-mediated TAR trans-activation which occurs in HIV infected cells.

TAT/TAR trans-activation can be conveniently assayed by placing the human placental alkaline phosphatase gene (PAP) under the regulatory control of the HIV-1 LTR sequences, which contain enhancer, promoter, and tar elements. A plasmid containing the HIV-1 LTR, pHIVCAT-0 (S. Feng, E.C. Holland, *Nature* 334, 165 (1988)), contains HIV U3 in its entirety and R up through position +78 (a HindIII site). Digestion of this plasmid with a combination of HindIII and AatII releases the CAT cassette along with the SV40 sequences responsible for the processing of the RNA. A second plasmid, pSV2Apap, contains the PAP cassette with eukaryotic processing signals, under the transcriptional control of an SV40 promoter. P. Henthron, P. Zervos, N. Raducha, H. Harris, T. Kadesch, *Proc. Natl. Acad. Sci. USA* 85, 6342 (1988). The PAP cassette and processing sequences were released from the plasmid by digestion with HindIII and AatII. A new plasmid, pHIVPAP, was created by ligating the HindIII/AatII fragment containing the HIV-1 LTR and vector sequences from pHIVCAT-0, to the HindIII/AatII PAP cassette from pSV2Apap.

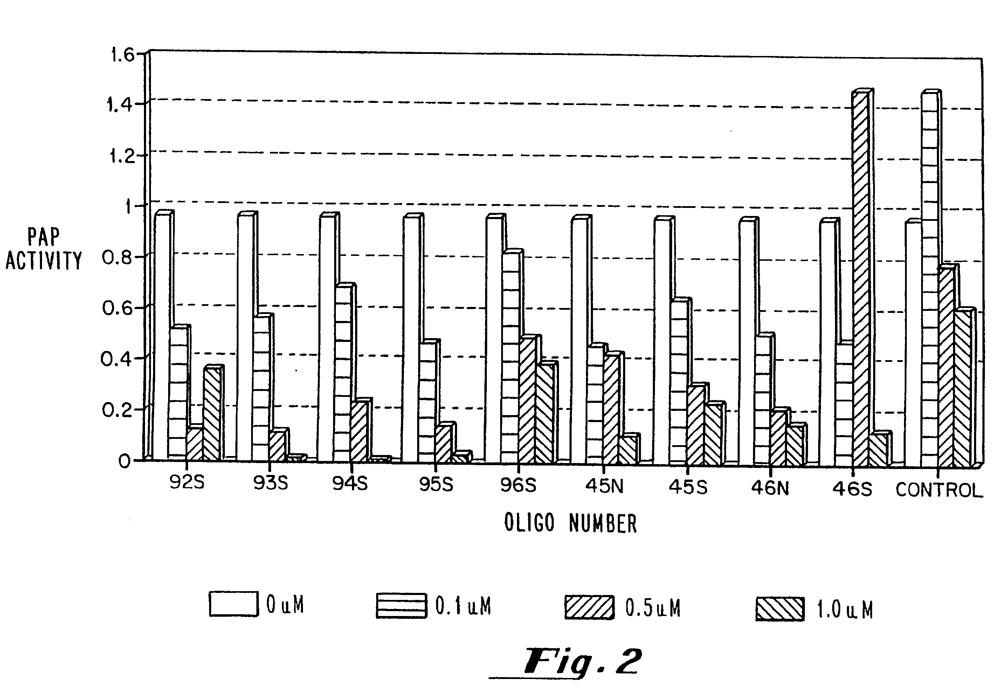

To test the activity of oligonucleotides and oligonucleotide analogs, pcDEBtat and PHIVPAP were co-transfected into HeLa cells by calcium/phosphate precipitation. The effects of oligonucleotides and oligonucleotide analogs was determined as follows. HeLa cells were split 1:8 into 6-well dishes the day prior to the transfections. For each dish, 1 µg of pHIVPAP and 12 µg of pcDEBtat were precipitated in 500 µl of HBS and 32 µl of 2.5 M CaCl₂. The CaPO₄ precipitate was divided evenly between the 6 wells. Oligonucleotides or oligonucleotide analogs were precipitated in the same manner and added to wells at the concentrations indicated on the figures (the volume is 1.5 ml of media per well). The precipitate was allowed to sit on the cells for 20 minutes then complete media was added and the cells were incubated for an additional 4 hours. The cells were then shocked with 10% glycerol in HBS. For figure 2 only, oligonucleotide was added back to the media following the transfection at a 10 fold higher dose per well. After 48 hours cells were harvested and protein and PAP assays performed as described by Henthorn et al. P. Henthorn, P. Zervos, N. Raducha, H. Harris, T. Kadesch, *Proc. Natl. Acad. Sci. USA 85, 6342* (1988) with the following modifications. The cells were harvested in 0.5 ml of TBS, of which 0.1 mls were used for use in the protein assay. The remaining 0.4 mls of cell suspension was pelleted then resuspended in 50 µl TBS. Endogenous phosphatases were inactivated by heating the cells at 65°C for 30 min. The heat stable human placental alkaline phosphatase activity was assayed by the addition of PNPP (0.5 ml, 5 mM PNPP) to the cell suspension, which was then incubated at 37°C. Activity was determined at 30 minute intervals using 150 µl aliquots of the reaction mixture and measuring absorbance at 405 nm with a Titertek Multiscan MCC\340 ELISA plate reader. The PAP activity was normalized to the total protein in each well as determined by Bio-Rad protein assay, in which 1/5 of the harvested cells in TBS(0.1 µl) were added to 30 µl of Bio-Rad Protein Reagent, then incubated for 10 minutes at room temperature, followed by measurement of absorbance at 595 nm using the Titertek plate reader.

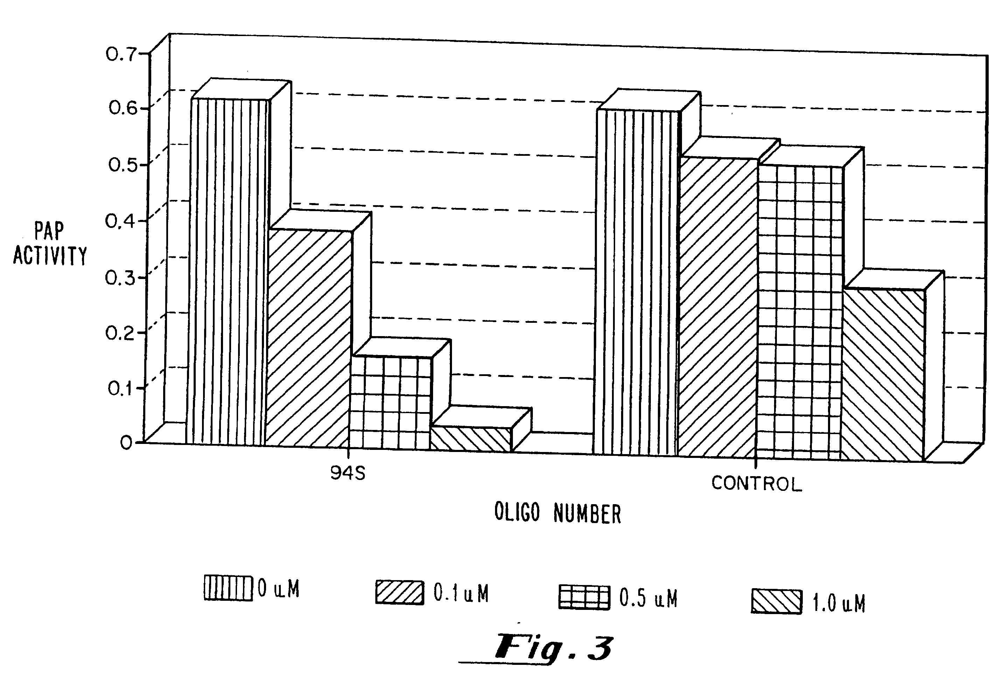

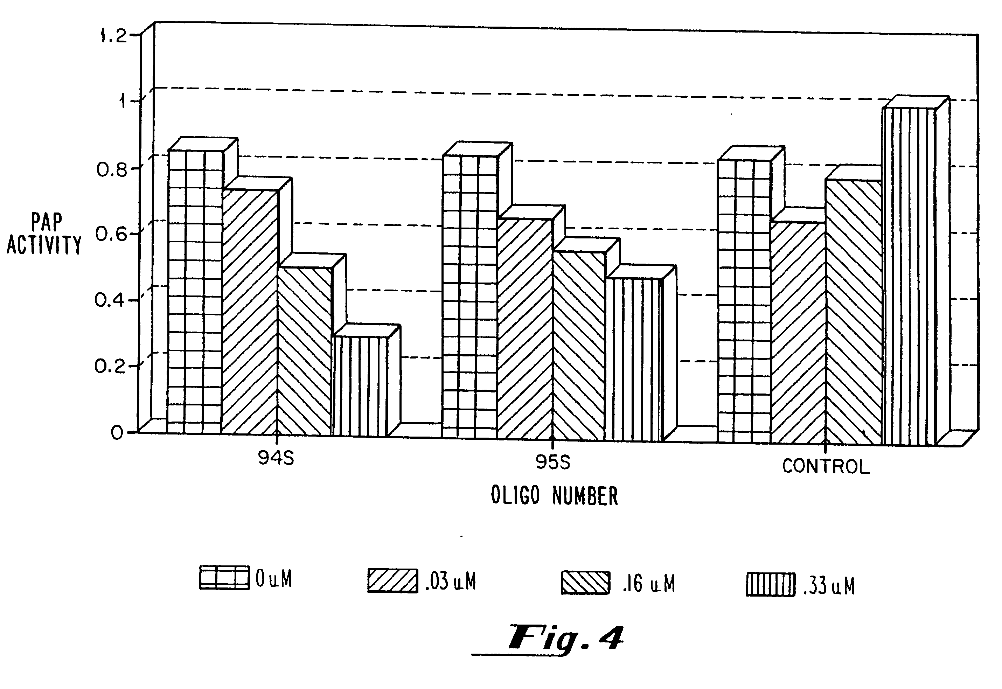

Cells were treated with the following oligonucleotides and oligonucleotide analogs: 
# | Sequence 5'--3'
91 | TCCCAGGC
92 | GTCTAACCAGAGAGACCC
93 | CAGATCTGGTCTAACCAGAGAGACCC
94 | GCTCCCAGGCTCAGATCT
95 | GCCAGAGAGCTCCCAGGCTCAGATCT
96 | GCCAGAGAGCTCCCAGGC
45 | GCTTAAGCAGTGGGTTCCCT
46 | CTTTATTGAGGCTTAAGCAG
Control oligo | CGACTCCGTGCTGGCTCTGA (RANDOM SEQUENCE)
 In Figures 2-4, the oligonucleotide or analog is numbered, followed by a letter (S or N), where oligonucleotides containing phosphodiester linkages are followed by "N" and oligonucleotide analogs containing all phosphorothioate linkages are followed by an "S". The results shown in Figures 2-4 represent three independent experiments.

### EXAMPLE 2. The HIV CAR element

One of the regulatory events in the life cycle of the human immunodeficiency virus is accumulation of the large virion structural RNA's which are accumulated at the expense of the shorter regulatory mRNA's. In essence, the virus uses much of the same RNA material to encode each set of proteins. If the RNA's are more extensively spliced, the regulatory proteins are produced. If the RNA's are less extensively spliced, the structural proteins are produced. W.A. Haseltine, F. Wong-Staal, *Scientific American* October, 52 (1988). These events are regulated by a protein known as rev, which a product of the rev gene. Rev's function is to enhance the transport of RNA from the nucleus of the cell to the cytoplasm. In the absence of rev, the mRNA's stay in the nucleus of the cell, where they are subject to splicing enzymes which convert them to mRNA's which encode regulatory proteins. In the presence of rev, the mRNA's are transported to the cytoplasm with less splicing. The resulting longer mRNA's encode structural proteins.

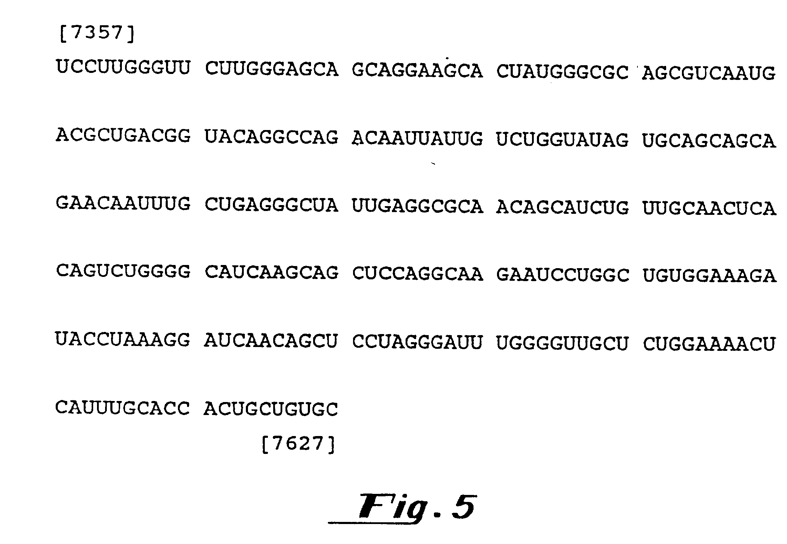

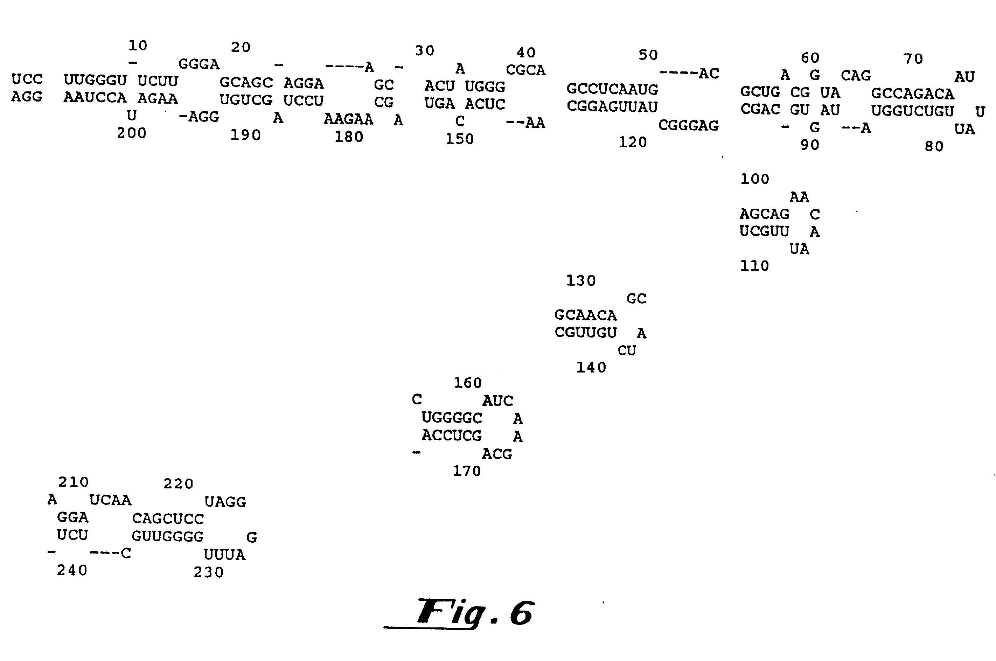

Rev functions by binding to an RNA structural element known as the CAR element. E.T. Dayton, D.M. Powell, A.I. Dayton, *Science* 246, 1625 (1989). This structural element has also been referred to as the rre (rev-responsive element). The functional RNA has been localized to a 269 bp region in the env RNA with the coordinates 7358-7627. L. Ratner, W. Haseltine, R. Patarca, K.J. Livak, B. Starcich, S.F. Josephs, *Nature 313,* 277 (1985). The sequence is shown in Figure 5. For convenience, this structure is referred to as the CAR element. The secondary structure of the CAR element is currently not known with certainty. However, it is possible to predict the secondary structure of the CAR element using computer programs commonly used by those skilled in the art such as the program of Zuker. M. Zuker, *Science* 244, 48 (1989). The result of such an analysis yields the result shown in Figure 6. Each of the stem loop structures shown in Figure 6 have the potential to interact with the rev gene product and each can be bound by oligonucleotides or oligonucleotide analogs as part of this invention. It is by no means certain that the structures predicted by the computer program and illustrated in Figure 6 are correct or exhaustive. This does not restrict the practice of this invention for the CAR element structure, however. In this, and all other cases where the actual RNA structure is uncertain, the invention can be practiced by preparing a series of oligonucleotides or oligonucleotide analogs which are complementary to the sequence, where the oligonucleotides or oligonucleotide analogs are designed with the constraints and with the considerations set forth herein.

Assays to measure the normal function of the rev gene product can be performed according to published procedures. E.T. Dayton, D.M. Powell, A.I. Dayton, *Science* 246, 1625 (1989); Dayton et al., J. Acq. Immune Deficiency Syndromes l, 441, 1988. Vectors which express HIV mRNA in cells under regulatory control of a variety of promoters are transfected into cells along with a vector which expresses the rev protein. When rev functions normally to facilitate the transport of mRNA to the cytoplasm, the transported mRNA's encode the gag protein, which is detected by an immunoabsorbant assay. When oligonucleotides or oligonucleotide analogs interfere with this process, a decrease in the production of gag protein is measured. The reagents needed to conduct these experiments are available from the National Institutes of Health (Aids Research and Reference Reagent Program, 1990 catalog, National Institute of Allergy and Infectious Diseases).

The effects of oligonucleotides and oligonucleotide analogs will be determined by adding the compounds directly to the transfection mixture or by adding the compounds to the media at various times and concentrations following transfection, followed by the assay at 24-48 hours post-transfection. The following oligonucleotides and analogs will be studied.

Sequence 5'--3'
GTGCAAATGAGTTTTCCAGA
GCAACCCCAAATCCCCAGGA
GCTGTTGATCCTTTAGGTAT
CTTTCCACAGCCAGGATTCT

TGCCTGGAGCTGCTTGATGC
CCCAGACTGTGAGTTGCAAC
AGATGCTGTTGCGCCTCAAT
AGCCCTCAGCAAATTGTTCT
GCTGCTGCACTATACCAGAC
AATAATTGTCTGGCCTGTAC
CGTCAGCGTCATTGACGCTG
CGCCCATAGTGCTTCCTGCT
GCTCCCAAGAACCCAAGGAA

### EXAMPLE 3. Inhibition of Frame Shifting: the HIV gag/pol Frameshift Region.

HIV and other retroviruses synthesize a protein which encodes a reverse transcriptase, pol, as part of a fusion with a structural protein known as gag. The virus also encodes a sequence-specific protease which cleaves between the gag and pol domains of the fusion protein to release free pol. In all retroviruses examined to date the genetic sequence of the gag-pol mRNA precludes direct translation of the mRNA into a gag-pol fusion protein. Either there is an in-frame termination codon between the gag and pol domains on the mRNA, or the gag and pol sequences are not in the same reading frame of the message. T. Jacks, M.D. Power, F.R. Masiarz, P.A. Luciw, P.J. Barr, H.E. Varmus, *Nature 331, 280* (1988). In the case of HIV, the pol reading frame is -1 relative to gag. In order to express a fusion protein the ribosome "frame shifts" at the junction between the gag and pol regions on the mRNA and continues translation in the reading frame of pol until completion of synthesis of the fusion protein.

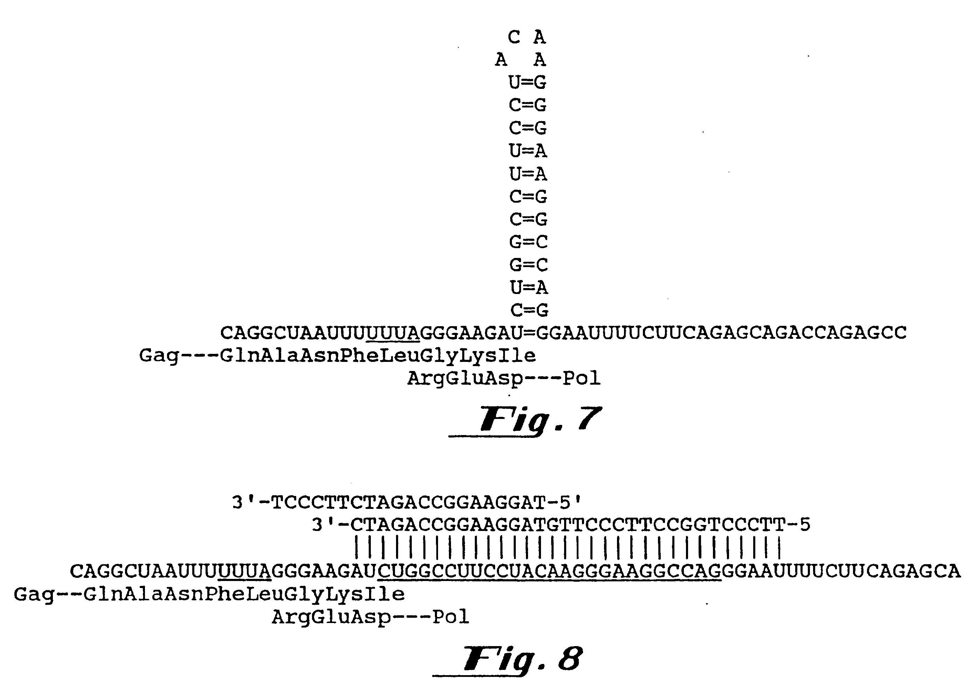

In HIV and other retroviruses, near the site of frame shifting, there is a potential for the formation of significant RNA secondary structure. A computer predicted structure for HIV-1 is illustrated in Figure 7. The potential formation of RNA secondary structures near the sites of ribosomal frame shifting exists in a number of viral gag-pol fusions. T. Jacks, K. Townsley, H. E. Varmus, J. Majors, Proc. *Natl. Acad. Sci. U. S.* A. 84, 4298 (1987); T. Jacks, M.D. Power, F.R. Masiarz, P.A. Luciw, P.J. Barr, H.E. Varmus, *Nature* 331, 280 (1988); and I. Brierley, P. Digard, S.C. Inglis, *Cell 57, 537 (1989).* It is now discovered that targeting the region between the HIV gag and pol genes with antisense oligonucleotides and oligonucleotide analogs is effective in modulating the expression of the gag-pol fusion protein. Figure 7 depicts the mRNA region of interest in the instant example, including the predicted stem-loop structure near the site of frameshifting and the predicted amino acid sequence of the gag and gag-pol fusion proteins near the frameshift site. Figure 8 depicts the sequences of two representative antisense oligonucleotides or oligonucleotide analogs which would be expected to modulate ribosomal frameshifting and translation of the target mRNA. By binding to this site by any of the following methods, it is possible to perturb the normal course of gene expression and as a result, inhibit the virus.

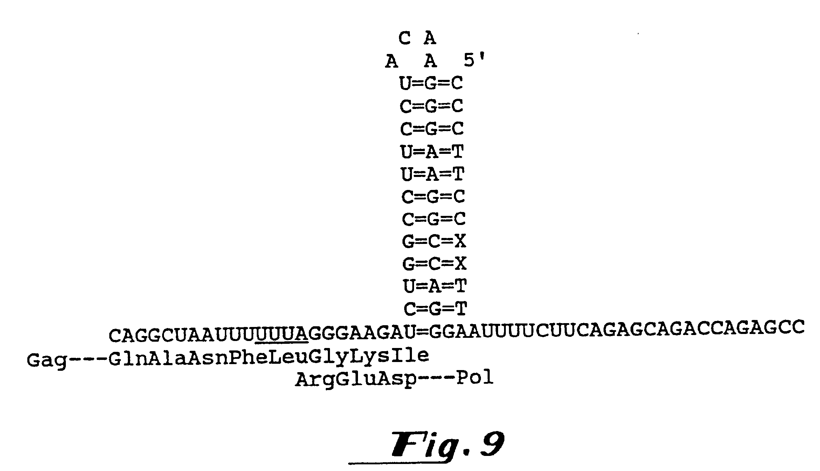

It is now discovered that compounds which specifically bind to the gag-pol frameshift region and interfere with translation and/or frameshifting are believed to have activity as therapeutic agents for retroviral infection. It is intended that all retroviruses which have RNA secondary structures at the gag-pol junctions fall within the spirit and scope of this invention. Different strains and types of retroviruses will have different gag-pol junctions with different secondary structures. This invention can be practiced on different strains or types of retroviruses by changing the sequence of the oligonucleotide or oligonucleotide analog to complement the structure of the alternative strain or type of retrovirus. Cells will be treated with the following oligonucleotides or oligonucleotide analogs: 
Sequence 5'--3'
TAGGAAGGCCAGATCTTCCCT
AAGAAAATTCCCTGGCCTTCCCTTGTAGGAAGGCCAG
TGCTCTGAAGAAAATTCCCT
TCTGAAGAAAATTCCCTGGC
 
GAAGAAAATTCCCTGGCCTT
CTGGCCTTCCCTTGTAGGAA
GCCTTCCCTTGTAGGAAGGC
TTCCCTTGTAGGAAGGCCAG
CCTTGTAGGAAGGCCAGATC
TGTAGGAAGGCCAGATCTTC
 It is also disclosed that the gag-pol RNA structure can be perturbed by binding to it by triple strand formation (see Figure 9). In contrast to heteroduplex formation, where the RNA secondary structure is broken by an invading strand, triple strand formation generally preserves the existing RNA duplex hydrogen bonding pattern, but binds in a helical groove with additional hydrogen bonds. Oligonucleotides of the following sequence will be tested: 
Sequence 5'--3'
CCCTTCCXXTT
CCTTCCXXT
GGGAAGGXXAG
GGAAGGXXA
 where X is any heterocyclic base containing a hydrogen bond acceptor. The assay for translation and ribosomal frame shifting has been previously described in T. Jacks, M.D. Power, F.R. Masiarz, P.A. Luciw, P.J. Barr, H.E. Varmus, Nature 331, 280 (1988). The assay will be performed as described with the addition of the above oligonucleotides and oligonucleotide analogs.

## Claims

1. A method of modulating the expression of a gene comprising:
selecting a portion of RNA coded by the gene, said RNA portion having subportions forming a secondary structure; and
contacting said RNA with oligonucleotide or oligonucleotide analog which can bind with at least one of the subportions of the RNA portion.

2. The method of claim 1 wherein said binding disrupts the secondary structure of the RNA portion to effect said modulation.

3. The method of claim 1 wherein the gene expression is suspected to give rise to a diseased state in an organism.

4. The method of claim 1 wherein said secondary structure is a loop.

5. The method of claim 1 wherein said gene is of a virus or retrovirus.

6. The method of claim 1 wherein said oligonucleotide or oligonucleotide analog can bind with at least about 6 subunits of said subportion.

7. The method of claim 1 wherein said oligonucleotide or oligonucleotide analog can bind with from about 8 to about 50 subunits of said subportion.

8. The method of claim 1 wherein said oligonucleotide or oligonucleotide analog can bind with from about 10 to about 20 subunits of said subportion.

9. The method of claim 1 wherein said oligonucleotide or oligonucleotide analog forms a duplex structure with said subportion.

10. The method of claim 1 wherein said oligonucleotide or oligonucleotide analog and said selected portion of RNA form a triplex.

11. The method of claim 1 wherein said binding alters ribosomal frame shifting.

12. The method of claim 1 wherein said RNA portion is coded by at least a part of the TAR element of HIV.

13. The method of claim 1 wherein said RNA portion is coded by at least a part of the CAR element of HIV.

14. The method of claim 1 wherein said RNA portion is coded by at least a part of the gag-pol element of HIV.

15. An oligonucleotide or oligonucleotide analog which can bind with a subportion of an RNA which is capable of forming a secondary structure.

16. The oligonucleotide or oligonucleotide analog of claim 15 having at least about 6 subunits.

17. The oligonucleotide or oligonucleotide analog of claim 15 having from at least about 8 to about 50 subunits.

18. The oligonucleotide or oligonucleotide analog of claim 15 having from at least about 10 to about 50 subunits.

19. The oligonucleotide or oligonucleotide analog of claim 15 capable of forming a duplex structure with said subportion.

20. The oligonucleotide or oligonucleotide analog of claim 15 capable of forming a triplex with said secondary structure.

21. The oligonucleotide or oligonucleotide analog of claim 15 in a pharmaceutically acceptable carrier.

22. A method of treating an animal suspected of having a disease characterized by expression of a gene coding for an RNA having a secondary structure comprising contacting the animal with an oligonucleotide or oligonucleotide analog which can bind with said secondary structure or any subportion thereof.

23. The method of claim 22 wherein said binding disrupts the secondary structure of the RNA portion to effect said modulation.

24. The method of claim 22 wherein said gene is of a virus or retrovirus.

25. The method of claim 22 wherein said oligonucleotide or oligonucleotide analog can bind with at least about 6 subunits of said subportion.

26. The method of claim 22 wherein said oligonucleotide or oligonucleotide analog can bind with from about 8 to about 50 subunits of said subportion.

27. The method of claim 22 wherein said oligonucleotide or oligonucleotide analog can bind with from about 10 to about 20 subunits of said subportion.

28. The method of claim 22 wherein said oligonucleotide or oligonucleotide analog forms a duplex structure with said subportion.

29. The method of claim 30 wherein said oligonucleotide or oligonucleotide analog and said RNA forms a triplex.

30. A method of treating an animal suspected of having HIV infection comprising administering to the animal an oligonucleotide or oligonucleotide analog capable of specifically hybridizing with the secondary structure or any subportion of the RNA coded by the TAR, CAR or gag-pol element.

31. The method of claim 30 wherein said animal is a human.
